# EUROPÄISCHE PATENTSCHRIFT

(11) **EP 2 100 871 B1**
(45) Veröffentlichungstag und Bekanntmachung des Hinweises auf die Patenterteilung: **19.12.2012**
(21) Anmeldenummer: 09003463.8
(22) Anmeldetag: 10.03.2009
(51) Int. Cl.: C07C 51/00, C07C 59/08

(54) **Katalytisches Verfahren zur Gewinnung von Milchsäure aus nachwachsenden Rohstoffen**
Catalytic method for preparing lactic acid from renewable raw materials
Procédé catalytique destiné à la production d'acide lactique à partir de matières premières renouvellables

(30) Priorität: 10.03.2008 DE 102008013474
(43) Veröffentlichungstag der Anmeldung: 16.09.2009
(73) Patentinhaber: Fraunhofer-Gesellschaft zur Förderung der angewandten Forschung e.V., 80686 München (DE)
(72) Erfinder: Danzig, Joachim, 46159 Oberhausen (DE); Fastabend, Anna, 47057 Duisburg (DE); Grän-Heedfeld, Jürgen, 46562 Voerde (DE); Kraft, Axel, 45739 Oer-Erkenschwick (DE)
(74) Vertreter: Weishäupl, Michael

(56) Entgegenhaltungen:
- EP-A- 0 523 015
- EP-A2- 0 523 014
- MARIS ET AL: "Hydrogenolysis of glycerol over carbon-supported Ru and Pt catalysts" JOURNAL OF CATALYSIS, ACADEMIC PRESS, DULUTH, MN, US, Bd. 249, Nr. 2, 4. Juli 2007 (2007-07-04), Seiten 328-337, XP022142438 ISSN: 0021-9517

## Beschreibung

Die vorliegende Erfindung betrifft ein katalytisches Verfahren zur Gewinnung von chemischen Grundstoffen aus nachwachsenden Rohstoffen.

Insbesondere betrifft die vorliegende Erfindung ein katalytisches Verfahren zur Herstellung von 2-Hydroxypropansäure (Milchsäure) bzw. deren Salzen (Lactaten), vorzugsweise ausgehend von Polyalkoholen (Polyolen) oder Polyhydroxyaldehyden, wie insbesondere Glycerinen (z. B. 1,2,3-Propantriol oder dessen Oligomeren bzw. Polyglycerinen) oder Zuckern und Zuckerderivaten (z. B. Mono-, Di-, Oligo- und Polysacchariden).

Gemäß einer bevorzugten Ausführungsform betrifft die vorliegende Erfindung ein zur Dehydrogenierung mehrwertiger Alkohole (Polyalkohole bzw. Polyole) geeignetes katalytisches Verfahren, welches aus biologischen, nachwachsenden Rohstoffquellen die chemischen Grundstoffe 2-Hydroxypropansäure (Milchsäure) bzw. Milchsäuresalze (Lactate) und Wasserstoff erzeugt, insbesondere wobei pro Molekül Milchsäure(salz) ein Molekül Wasserstoff freigesetzt wird.

Milchsäure, synonym auch als 2-Hydroxypropansäure oder 2-Hydroxypropionsäure bezeichnet, ist eine chemische Verbindung mit der Halbstrukturformel CH₃-CH(OH)-COOH, welche ein wichtiges Zwischenprodukt im menschlichen Stoffwechsel darstellt und beispielsweise ein Produkt beim Abbau von Zuckern durch eine anaerobe Glycolyse darstellt. Die Salze der Milchsäuren werden als Lactate bezeichnet. Im menschlichen Organismus kommt Milchsäure in Form von sogenannter L-(+)-Milchsäure beispielsweise im Schweiß, Blut, Muskelserum, der Niere und der Galle vor.

Milchsäure stellt eine relevante, aus Biomasse ableitbare Grundchemikalie dar. Beispielsweise dient Milchsäure als Grundbaustein für Lösungsmittel oder oberflächenaktive Substanzen, Milchsäure bzw. deren Salze, die Lactate, finden darüber hinaus in verschiedenen Produkten Anwendung, beispielsweise in Kosmetika, Nahrungsmitteln als Nahrungsmitteladditive, Agrochemikalien oder dergleichen.

Auch bei der Herstellung von sogenannten Biopolymeren auf Basis von Polymilchsäuren (Polylactiden bzw. PLA) gewinnt Milchsäure (2-Hydroxypropansäure) zunehmend an Bedeutung. PLA kann aus dem natürlichen Rohstoff Milchsäure über unterschiedliche Synthesewege erzeugt werden.

Zu weitergehenden Einzelheiten zur Anwendbarkeit von Milchsäure bzw. Lactaten und den hieraus erhältlichen Produkten kann beispielsweise auf den Übersichtsartikel von R. Datta et al. "Lactic acid: recent advances in products, processes and technologies - a review" in: J. Chem. Technol. Biotechnol. 81: 1119-1129 (2006), verwiesen werden.

Die Milchsäureherstellung im Wege der Fermentation ist heute Stand der Technik und versorgt die Lebensmittel-, Pharma- und Kosmetikindustrie mit diesem Grundstoff. Inzwischen geht die fermentative Herstellung im überwiegenden Fall von Zucker als Basisrohstoff aus.

Mit der fermentativen Herstellung von Milchsäure nach dem Stand der Technik sind jedoch eine Reihe von Nachteilen verbunden. So sind beispielsweise mit der Herstellung von Milchsäure im Wege der Fermentation nur relativ geringe Raum-Zeit-Ausbeute möglich, welche im allgemeinen im Bereich von nur 5 bis 30 g/(l·h) liegen. Darüber hinaus ist die Abtrennung der Milchsäure aus dem Fermentationsmilieu mit einem nicht unerheblichen Aufwand verbunden. Weiterhin sind die Fermentationsbedingungen in sehr engen Grenzen einzuhalten, so daß eine aufwendige prozeßtechnische Kontrolle erforderlich ist. Bei der herkömmlichen heterofermentativen Milchsäurefermentation entstehen zudem in vielen Fällen auch unerwünschte Nebenprodukte, welche zum Teil nicht weiterverwertbar sind oder nur schwierig von dem gewünschten Produkt abzutrennen sind.

EP0523015 offenbart ein katalytisches Verfahren zur Hydrierung von Glycerin, bei welchem sauerstoffhaltige Verbindungen mit 1 bis 3 Kohlenstoffatomen, in der Hauptsache 1,2-Propanediol und 1,2-Ethanediol und ein bisschen Milchsäure erzeugt werden. Bei Durchführung des Verfahrens wird Glycerin mit Wasserstoff in Kontakt gebracht und mit diesem in Gegenwart eines Kupfer und Zink enthaltenden Katalysators bei Temperaturen von mindestens 200°C zur Reaktion gebracht, in Abwesenheit basischer Verbindungen.

MARIS ET AL: "Hydrogenolysis of glycerol over carbon-supported Ru and Pt catalysts", JOURNAL OF CATALYSIS, 249(2), 2007, pp 328-337 offenbart ein katalytisches Verfahren zur Hydrogenolyse von Glycerin an Ruthenium- und Platinkatalysatoren, in Gegenwart von Natriumhydroxyd oder Calciumoxyd bei Temperaturen bis 200°C und einem Druck von 5 bar mit einer Selektivität in Bezug auf Milchsäure von 19 bis 65 Mol%.

EP0523014 offenbart ein katalytisches Verfahren zur Herstellung von Milchsäure aus Glycerin in Gegenwart eines Rutheniumkatalysators und Natriumhydroxyd wobei das Verfahren unter einem Absolutdruck von 130 bar und bei Temperaturen im Bereich von 230 bis 270°C, mit einer Selektivität in Bezug auf Milchsäure von 1.5 bis 23.5 Mol%, durchgeführt wird.

Der vorliegenden Erfindung liegt somit die Aufgabe zugrunde, ein Verfahren zur Gewinnung von chemischen Grundstoffen aus nachwachsenden Rohstoffen, insbesondere ein Verfahren zur Herstellung von 2-Hydroxypropansäure (Milchsäure) bzw. deren Salzen (Lactaten), bereitzustellen, welches die Nachteile der zuvor geschilderten, aus dem Stand der Technik bekannten Verfahren zumindest teilweise vermeidet oder aber wenigstens abschwächt.

Zur Lösung des zuvor geschilderten Problems schlägt die vorliegende Erfindung ein Verfahren gemäß Anspruch 1 vor; weitere, insbesondere vorteilhafte Ausgestaltungen sind Gegenstand der diesbezüglichen Verfahrensunteransprüche.

Gegenstand der vorliegenden Erfindung ist somit ein Verfahren zur Herstellung von 2-Hydroxypropansäure (Milchsäure) oder deren Salzen (Lactaten), bei dem wenigstens eine mindestens drei Kohlenstoffatome aufweisende organische Ausgangsverbindung, welche ausgewählt ist aus einem primären Alkohol der allgemeinen Formel (I') 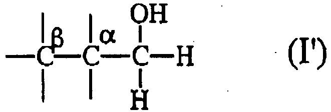 und/oder einem Aldehyd der allgemeinen Formel (I") 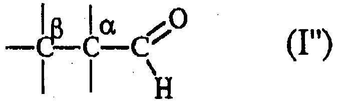 wobei der primäre Alkohol bzw. der Aldehyd eine Hydroxylgruppe in α-Position zur primären Alkoholfunktion bzw. zur Aldehydgruppe sowie eine Hydroxyl- oder Ethergruppe in β-Position zur primären Alkoholfunktion bzw. zur Aldehydgruppe aufweist,
in Gegenwart eines Übergangsmetallkatalysators zu 2-Hydroxypropansäure (Milchsäure) oder deren Salzen (Lactaten) umgesetzt wird.

Für den Fall, daß in den beiden oben bezeichneten Formeln (I') bzw. (I") eine Ethergruppe in β-Position zur primären Alkoholfunktion bzw. zur Aldehydgruppe vorhanden ist, kann diese Ethergruppe insbesondere durch einen Rest -OG dargestellt sein, wobei G einen organischen Rest bezeichnet, insbesondere wobei der organische Rest G ausgewählt sein kann aus einem oder mehreren der folgenden Reste: (A) einer Alkylgruppe; (B) einer Arylgruppe; (C) einer Arylalkylgruppe; (D) einer Alkylarylgruppe; (E) einer polyalkoholischen Gruppe (Polyolylgruppe), insbesondere ausgewählt aus (i) Mono-, Di- oder Poly-1,2,3-propantriolylgruppen (Mono-, Di- oder Polyglyceringruppen), vorzugsweise linearen, verzweigten und/oder cyclischen Mono-, Di- oder Polyglyceringruppen mit 1 bis 10 Glycerineinheiten, oder (ii) Resten, insbesondere C3- oder C6-Resten, von Zuckern oder Zuckerderivaten, vorzugsweise auf Hexosebasis.

Im allgemeinen ist das Übergangsmetall des Katalysators ausgewählt aus Metallen der Gruppen I B, II B, III B, IV B, V B, VI B, VII B und VIII B des Periodensystems der Elemente sowie deren Mischungen, Gemischen und Kombinationen, insbesondere aus den Metallen Kupfer, Cobalt, Nickel, Silber, Zink, Palladium, Ruthenium, Platin und/oder Rhodium.

In bevorzugter Ausflührungsform wird als Übergangsmetallkatalysator ein auf Kupfer basierter und/oder Kupfer enthaltender Katalysator, bevorzugt Cu⁰, eingesetzt. In erfindungsgemäß besonders bevorzugter Ausführungsform wird als Übergangsmetallkatalysator ein auf Kupfer basierter und/oder Kupfer enthaltender Katalysator, bevorzugt Cu⁰, in Kombination mit mindestens einem weiteren Metall, vorzugsweise in Kombination mit Zirconium, Cobalt, Nickel, Silber, Zink, Palladium, Ruthenium, Platin und/oder Rhodium, besonders bevorzugt in Kombination mit Zirconium, ganz besonders bevorzugt in Form von Cu⁰Zr^{IV}O₂, eingesetzt.

Grundsätzlich kann der Katalysator in Masse bzw. als solcher (z. B. in amorpher bzw, feinverteilter Form) oder aber in geträgerter Form, d. h. als sogenannter Trägerkatalysator auf geeigneten Trägermaterialien, eingesetzt werden.

Im allgemeinen wird im Rahmen des erfindungsgemäßen Verfahrens der Katalysator in aktivierter Form eingesetzt. Dabei kann die Aktivierung insbesondere unter Wasserstoffatmosphäre, insbesondere bei einem Absolutdruck von 1 bis 5 bar, vorzugsweise 2 bis 3 bar, durchgeführt werden. Im allgemeinen erfolgt die Aktivierung des Katalysators bei Temperaturen im Bereich von 150 bis 300 °C, vorzugsweise 180 bis 250 °C.

Üblicherweise wird der Katalysator nach Durchführung des erfindungsgemäßen Verfahrens von den erhaltenen Reaktionsprodukten abgetrennt und, gegebenenfalls nach zwischenzeitlicher Aufreinigung und/oder Aktivierung, nachfolgend rezykliert, d. h. wieder im erfindungsgemäßen Verfahren eingesetzt.

Üblicherweise wird das erfindungsgemäße Verfahren unter Inertgasatmophäre (z. B. Edelgas- und/oder Stickstoffatmosphäre) oder unter Wasserstoffatmosphäre, insbesondere unter Wasserstoffatmosphäre, durchgeführt. Im allgemeinen wird das erfindungsgemäße Verfahren bei einem Inertgaspartialdruck, insbesondere Wasserstoffpartialdruck, von weniger als 20 bar, vorzugsweise im Bereich von 0,5 bis < 20 bar, bevorzugt 5 bis 15 bar, besonders bevorzugt 8 bis 10 bar, durchgeführt. Partialdrucke oberhalb von 20 bar sind erfindungsgemäß weniger geeignet, da hier konkurrierende Hydrierungsreaktionen einsetzen können.

Im allgemeinen wird das erfindungsgemäße Verfahren daher unter einem Absolutdruck von weniger als 20 bar durchgeführt. Üblicherweise kann das erfindungsgemäße Verfahren unter einem Absolutdruck im Bereich von 0,1 bis < 20 bar, bevorzugt 1 bis 10 bar, durchgeführt werden.

Was die Verfahrenstemperaturen anbelangt, so wird das erfindungsgemäße Verfahren vorteilhafterweise bei Temperaturen oberhalb von 50 °C und/oder unterhalb von 300 °C durchgeführt. Vorzugsweise wird das erfindungsgemäße Verfahren im Bereich von 80 bis 250 °C, insbesondere 100 bis 200 °C, bevorzugt 120 bis 190 °C, besonders bevorzugt 140 bis 180 °C, durchgeführt.

Im allgemeinen erfolgt die Durchführung des Verfahrens unter basischen Reaktionsbedingungen, insbesondere bei pH-Werten im Bereich von 8 bis 14, insbesondere bei einem pH-Wert > 8, bevorzugt bei einem pH-Wert > 9, besonders bevorzugt bei einem pH-Wert > 10. In diesem Fall werden die Salze der Milchsäure, die Lactate, als Reaktionsprodukte erhalten.

Im allgemeinen wird das erfindungsgemäße Verfahren in vorzugsweise wäßriger Lösung unter heterogener Katalyse durchgeführt.

Das erfindungsgemäße Verfahren kann in beliebigen Reaktoren betrieben werden, insbesondere solchen, die eine ausreichende Durchmischung gewährleisten und zudem eine Druckbeaufschlagung ermöglichen.

In besonders bevorzugter Ausführungsform kann das erfindungsgemäße Verfahren in Gegenwart mindestens eines Promotors, insbesondere in Gegenwart eines Komplexbildners für die hergestellte 2-Hydroxypropansäure (Milchsäure) oder deren Salze (Lactate), vorzugsweise in Gegenwart eines Erdalkalimetalls, besonders bevorzugt Calcium und/oder Magnesium, durchgeführt werden. Dabei kann der Promotor beispielsweise in Kombination mit dem Katalysator vorliegen, insbesondere hierauf aufgebracht sein, oder aber in der Reaktionslösung befindlich sein.

Üblicherweise wird das erfindungsgemäße Verfahren in Abwesenheit weiterer Reaktionspartner durchgeführt, d. h. im Rahmen des erfindungsgemäßen Verfahrens wird nur das vorgenannte Ausgangsmaterial als solches katalytisch umgesetzt.

Für den Fall, daß als Ausgangsmaterial der primäre Alkohol der allgemeinen Formel (I') eingesetzt wird, erfolgt die Reaktion unter gleichzeitiger Dehydrogenierung bzw. Generierung von Wasserstoff. Hierbei wird im allgemeinen pro 1 Mol erzeugter 2-Hydroxypropansäure (Milchsäure) bzw. deren Salz (Lactat) 1 Mol Wasserstoff generiert.

Was die erfindungsgemäß eingesetzte Ausgangsverbindung anbelangt, so weist diese mindestens drei Kohlenstoffatome oder ein Mehrfaches hiervon auf, beispielsweise zwei, drei, vier oder mehr C3-Einheiten. Im Fall von Ausgangsverbindungen von mehr als einer C3-Einheit erfolgt die Umsetzung bzw. Reaktion unter Molekülspaltung.

Wie bereits zuvor ausgeführt, erfolgt die Durchführung des Verfahrens im allgemeinen unter basischen Reaktionsbedingungen. Dabei beträgt das molare Verhältnis von eingesetztem OH⁻ zu zu erzeugender Milchsäure bzw. Lactat mindestens 0,1 : 1, insbesondere mindestens 1 : 1, und variiert insbesondere im Bereich von 0,1 : 1 bis 10 : 1, insbesondere 1 : 1 bis 10 : 1, vorzugsweise 1,05 : 1 bis 5 : 1, besonders bevorzugt 1,1 : 1 bis 2 : 1, ganz besonders bevorzugt 1,1 : 1 bis 1,5 : 1.

Bezogen auf die umzusetzende Ausgangsverbindung, sollte das molare Verhältnis von eingesetztem OH⁻ zu umzusetzender C3-Einheit pro Ausgangsverbindung mindestens 0,1 : 1, insbesondere mindestens 1 : 1, und variiert insbesondere im Bereich von 0,1 : 1 bis 10 : 1, insbesondere 1 : 1 bis 10 : 1, vorzugsweise 1,05 : 1 bis 5 : 1, besonders bevorzugt 1,1 : 1 bis 2 : 1, ganz besonders bevorzugt 1,1 : 1 bis 1,5 : 1, variieren.

Das erfindungsgemäße Verfahren läßt sich mit hohen Raum-Zeit-Ausbeuten in bezug auf Milchsäure bzw. Lactat durchführen. Insbesondere lassen sich mit dem erfindungsgemäßen Verfahren Raum-Zeit-Ausbeuten in bezug auf Milchsäure bzw. deren Salz von mindestens 30 g/(l · h), insbesondere im Bereich von 30 bis 200 g/(l • h), realisieren.

Ein Vorteil des erfindungsgemäßen Verfahrens liegt darin, daß es mit einer guten bis hohen Selektivität in bezug auf Milchsäure bzw. Lactat durchgeführt werden kann. Üblicherweise wird das erfindungsgemäße Verfahren mit einer Selektivität in bezug auf Milchsäure bzw. Lactat, berechnet als prozentuales Verhältnis der gebildeten Molmenge Milchsäure bzw. Lactat zu der eingesetzten Molmenge an Ausgangsverbindung, von mindestens 50 Mol-%, insbesondere mindestens 55 Mol-%, vorzugsweise mindestens 60 Mol-%, durchgeführt.

Weiterhin wird das erfindungsgemäße mit guten bis hohen massebezogenen Umsätzen (Stoffumsätzen) durchgeführt. Im allgemeinen wird das erfindungsgemäße Verfahren mit einem massenbezogenen Umsatz (Stoffumsatz), berechnet als prozentuales Verhältnis der umgesetzten Menge (Masse) an Ausgangsverbindung zu der eingesetzten Menge (Masse) an Ausgangsverbindung, von mindestens 80 %, insbesondere mindestens 85 %, vorzugsweise mindestens 90 %, besonders bevorzugt mindestens 95 %, durchgeführt.

Des weiteren kann das erfindungsgemäße Verfahren mit hohen Ausbeuten durchgeführt werden. Im allgemeinen wird das erfindungsgemäße Verfahren mit einer massenbezogenen Ausbeute, berechnet als prozentuales Verhältnis der erhaltenen Menge (Masse) an Milchsäure bzw. Lactat zu der eingesetzten Menge (Masse) an Ausgangsverbindung, von mindestens 50 %, insbesondere mindestens 60 %, vorzugsweise mindestens 70 %, besonders bevorzugt mindestens 75 %, durchgeführt.

Erfindungsgemäß bevorzugt ist es, wenn die für das erfindungsgemäße Verfahren als Edukt eingesetzte Ausgangsverbindung der allgemeinen Formel (Ia) 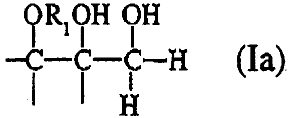 oder der allgemeinen Formel (Ib) 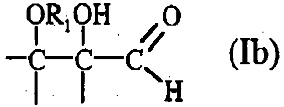 entspricht, wobei in den Formeln (Ia) und (Ib) der Rest R₁, unabhängig voneinander, jeweils Wasserstoff oder einen organischen Rest, vorzugsweise Wasserstoff, bezeichnet. Dabei kann der organische Rest insbesondere ausgewählt sein aus einem oder mehreren der folgenden Reste:
- einer Alkylgruppe;
- einer Arylgruppe;
- einer Arylalkylgruppe;
- einer Alkylarylgruppe;
- einer polyalkoholischen Gruppe (Polyolylgruppe), insbesondere ausgewählt aus (i) Mono-, Di- oder Poly-1,2,3-propantriolylgruppen (Mono-, Di- oder Polyglyceringruppen), vorzugsweise linearen, verzweigten und/oder cyclischen Mono-, Di- oder Polyglyceringruppen mit 1 bis 10 Glycerineinheiten, oder (ii) Resten, insbesondere C3- oder C6-Resten, von Zuckern oder Zuckerderivaten, vorzugsweise auf Hexosebasis.

Erfindungsgemäß ebenfalls bevorzugt ist es, wenn die für das erfindungsgemäße Verfahren als Edukt eingesetzte Ausgangsverbindung der allgemeinen Formel (Ia') 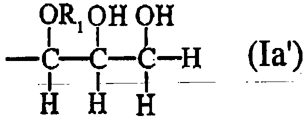 oder der allgemeinen Formel (Ib') 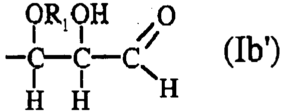 entspricht, wobei in den Formeln (Ia') und (Ib') der Rest R₁ die zuvor angegebene Bedeutung hat.

Erfindungsgemäß gleichermaßen bevorzugt ist es, wenn die für das erfindungsgemäße Verfahren als Edukt eingesetzte Ausgangsverbindung der allgemeinen Formel (Ia") 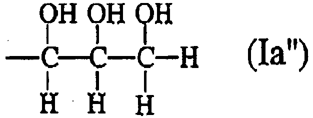 oder der allgemeinen Formel (Ib") 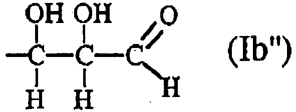 entspricht.

In den vorstehenden allgemeinen Formeln (I'), (I"), (Ia), (Ib), (Ia'), (Ib'), (Ia") und (Ib") bezeichnen die freien Valenzen, jeweils unabhängig voneinander, insbesondere Wasserstoff; Alkyl; gegebenenfalls substituiertes Aryl, insbesondere Phenyl, Hydroxyphenol und Alkoxyphenyl, insbesondere Methoxyphenyl; Arylalkyl; Alkylaryl; Halogen, vorzugsweise Fluor.

Erfindungsgemäß ebenfalls bevorzugt ist es, wenn die für das erfindungsgemäße Verfahren als Edukt eingesetzte Ausgangsverbindung der allgemeinen Formel 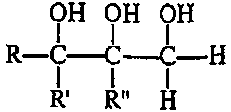 entspricht, wobei die Reste R, R' und R", jeweils unabhängig voneinander, Wasserstoff; Alkyl; gegebenenfalls substituiertes Aryl, insbesondere Phenyl, Hydroxyphenol und Alkoxyphenyl, insbesondere Methoxyphenyl; Arylalkyl; Alkylaryl; Halogen, vorzugsweise Fluor, bezeichnen.

Erfindungsgemäß bevorzugt ist es, wenn die für das erfindungsgemäße Verfahren als Edukt eingesetzte Ausgangsverbindung der allgemeinen Formel 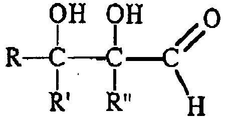 entspricht, wobei die Reste R, R' und R", jeweils unabhängig voneinander, Wasserstoff; Alkyl; gegebenenfalls substituiertes Aryl, insbesondere Phenyl, Hydroxyphenol und Alkoxyphenyl, insbesondere Methoxyphenyl; Arylalkyl; Alkylaryl; Halogen, vorzugsweise Fluor, bezeichnen.

Gemäß einer besonders bevorzugten Ausführungsform der vorliegenden Erfindung ist die einzusetzende Ausgangsverbindung insbesondere ausgewählt aus
(i) 1,2,3-Propantriol (Glycerin) und dessen linearen, verzweigten oder cyclischen Oligomeren (Oligoglycerinen) und Polymeren (Polyglycerinen);
(ii) Glycerinaldehyd (2,3-Hydroxypropionaldehyd);
(iii) Zuckern und Zuckeralkoholen, insbesondere Mono- und Disacchariden, bevorzugt auf Basis von Hexosen, ganz besonders bevorzugt Sucrose (Saccharose), Glucose, Fructose, Galactose, Lactose und Maltose, sowie daraus ableitbaren Zuckeralkoholen, insbesondere Sorbitol und Mannitol;
(iv) Polysacchariden, insbesondere auf Basis von Hexosen, bevorzugt Stärke und Cellulose;
sowie deren Gremischen.

Im Rahmen des erfindungsgemäßen Verfahrens wird neben den zuvor genannten Produkten (d. h. Milchsäure bzw. Lactate und gegebenenfalls Wasserstoff) als Nebenprodukt 1,2-Propandiol gebildet, insbesondere wenn von Glycerin bzw. den vorgenannten Glycerinderivaten oder Glycerinaldehyd als Ausgangsmaterial ausgegangen wird. Das als Nebenprodukt erzeugte 1,2-Propandiol kann nachfolgend ohne weiteres zu Milchsäure bzw. Lactaten umgesetzt werden.

Im einfachsten Fall wird das erfindungsgemäße Verfahren mit den Ausgangsstoffen Glycerin (1,2,3-Propantriol) oder Glycerinaldehyd durchgeführt.

Im Fall von Glycerin als Ausgangsmaterial erfolgt die Reaktion gemäß folgender Reaktionsgleichung, wobei pro umgesetztem Molekül Glycerin bzw. pro gebildetem Molekül Milchsäure gleichzeitig ein Molekül Wasserstoff generiert wird, d. h. gleichzeitig eine Dehydrogenierung stattfindet (Kat. = Katalysator):

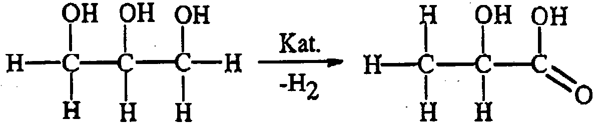

Im Fall von Glycerinaldehyd als Ausgangsmaterial erfolgt die Reaktion gemäß folgender Reaktionsgleichung, wobei im Unterschied zu Glycerin als Ausgangsstoff kein Wasserstoff regeneriert wird bzw. keine Dehydrogenierung stattfindet (Kat. = Katalysator):

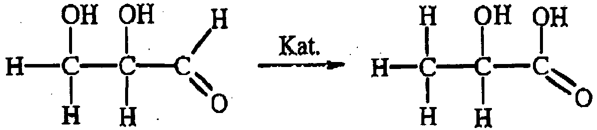

Im Fall von Polyalkoholen und Polyaldehyden mit mehreren C3-Einheiten erfolgt gleichzeitig eine Molekülspaltung, was beispielhaft anhand eines linearen Polyglycerins gemäß der folgenden Reaktionsgleichung dargestellt wird, wobei n beispielsweise Werte von 1 bis 10 annehmen kann (Kat. = Katalysator):

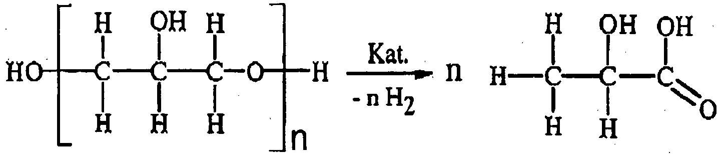

Bevorzugterweise können die eingesetzten Ausgangsverbindungen in Form von Roh- oder Abfallmaterialien eingesetzt werden, wie nachfolgend noch detailliert beschrieben.

So hat es sich beispielsweise als besonders vorteilhaft erwiesen, wenn die Ausgangsverbindung bzw. das Gemisch der Ausgangsverbindungen in Form von Roh- oder Abfallmaterialien eingesetzt wird. So lassen sich im Rahmen der vorliegenden Erfindung beispielsweise entsäuertes Rohglycerin (d. h. von Fett, Fettsäureestern und von freier Fettsäure befreites Glycerin beispielsweise aus der Biodieselherstellung oder der Oleochemie) oder Rohzuckergemische ohne vorangehende Aufreinigung im Rahmen des erfindungsgemäßen Verfahrens zum Einsatz bringen.

Daß im Rahmen des erfindungsgemäßen Verfahrens mit hoher Selektivität und hohen Umsätzen ausgehend von den zuvor spezifizierten Ausgangsverbindungen Milchsäure bzw. Lactate und gegebenenfalls Wasserstoff (in Abhängigkeit von der chemischen Natur der Ausgangsverbindungen) generiert wird, ist überraschend und für den Fachmann nicht zu erwarten. Denn, beispielsweise entsprechend der US 5 367 112 A, wäre vielmehr zu erwarten gewesen, daß die Ausgangsverbindung, insbesondere der Polyalkohol, beispielsweise Sorbitol, nur an einer oder an beiden primären Alkoholgruppen zu einer Carbonsäure oxidiert wird. Der erfindungsgemäß einzusetzende Reaktionsverlauf dagegen war für den Fachmann nicht zu erwarten.

Auch überraschend ist, daß - entgegen den Erwartungen des Fachmanns - die Reaktion mit guten Ausbeuten verläuft, auch wenn von Zuckern bzw. Zuckeralkoholen ausgegangen wird, ohne daß eine nennenswerte Karamelisierung der Ausgangsverbindung eintritt. Hier wäre vielmehr zu erwarten gewesen, daß wäßrige Lösungen von Zuckern, wie etwa Saccharose (Rübenzucker), bei den Temperaturen (z. B. zwischen 130 und 200 °C) nicht stabil sind und zur Karamelisierung neigen, was leicht durch Erwärmung einer wäßrigen Rohrzuckerlösung auf Siedetemperatur nachvollzogen werden kann. Daß dies im Rahmen des erfindungsgemäßen Verfahrensablaufs nicht eintritt, war daher nicht zu erwarten.

Weiterhin von besonderem Vorteil ist die Tatsache, daß im Rahmen des erfindungsgemäßen Verfahrens nachwachsende Rohstoffe als Ausgangsmaterialien zum Einsatz gebracht werden können.

Nachwachsende Rohstoffe werden vielfach in ihrer zukünftigen Bedeutung als Rohstoffbasis hervorgehoben. Als Substitut für petrochemische Rohstoffe gelten sie als Basis für neue Chemikalien, Pharmazeutika, Polymere oder dergleichen. Die Eignung nachwachsender Rohstoffe als Rohstoffe für industrielle Prozesse außerhalb der Nahrungsmittelproduktion ist essentiell von ihrer Verfügbarkeit und ihrem Marktpreis abhängig (sogenannte "*Large-scalecommodities*"). Aus diesem Grunde werden für die Entwicklung neuartiger Verfahren und Produkte zucker-, öl- und lignocellulosehaltige Rohstoffe herangezogen. Evaluierungen dieser Rohstoffbasen liegen bereits vor.

Die Erfindung ermöglicht sowohl die energetische als auch die stoffliche Nutzung nachwachsender Rohstoffe. Mit dem Verfahren können öl-, zucker- und stärkehaltige Rohstoffe umgewandelt werden, wie zuvor beschrieben bevorzugt 1,2,3-Propantriol (Glycerin), Oligomere des 1,2,3-Propantriols (Polyglycerine), alle Mono- und Disaccharide, insbesondere Sucrose, Glucose, Fructose, Galactose, Lactose und Maltose sowie daraus ableitbare Zuckeralkohole, insbesondere Sorbitol und Mannitol, sowie Polysaccharide, insbesondere Stärke und Cellulose.

Das erfindungsgemäße katalytische Verfahren wandelt diese aus nachwachsenden Rohstoffen ableitbaren Einsatzstoffe mit guter bis hoher Selektivität und hoher Raum-Zeit-Ausbeute in die chemischen Grundstoffe 2-Hydroxypropansäure (Milchsäure) und gegebenenfalls 1,2-Propandiol als Nebenprodukt (welches wiederum zu Milchsäure weiter umgesetzt werden kann) um, wobei pro Molekül Milchsäure ein Molekül Wasserstoff freigesetzt wird, sofern von primären Alkoholen ausgegangen wird.

Das erfindungsgemäße katalytische Verfahren ist sowohl auf primäre Alkohole anwendbar, was dem Stand der Technik entspricht, insbesondere jedoch erfindungsgemäß auf mehrwertige Alkohole und deren Oligomere. Gewisse Etherbindungen der Ausgangssubstanzen werden mit dem erfindungsgemäßen Verfahren überraschenderweise gespalten, so daß eine selektive Umsetzung zu den Produkten 2-Hydroxypropansäure (Milchsäure) und gegebenenfalls 1,2-Propandiol als Nebenprodukt erfolgt.

Für das erfindungsgemäße Verfahren können Katalysatoren eingesetzt werden, welche Kupfer, Zirconium und gegebenenfalls weitere Metalle enthalten, wobei der im wesentlichen kupferbasierte Katalysator nicht notwendigerweise auf Trägermaterial fixiert sein muß, sondern auch als sogenanntes Raney-Kupfer eingesetzt werden kann.

Die Besonderheit des erfindungsgemäßen Verfahrens liegt insbesondere darin, daß kostengünstige Produkte, insbesondere entsäuertes Rohglycerin (d. h. von Fett, Fettsäureestern und von freier Fettsäure befreites Glycerin beispielsweise aus der Biodieselherstellung oder der Oleochemie bzw. Fettspaltung) oder Aufschlüsse zuckerbasierter Rohstoffe (wie z. B. Sorbitol und Mannitol) oder Zuckergemische z. B. aus Glucose, Maltose und Fructose oder Polysaccharide mit hoher Ausbeute direkt zu den Wertprodukten umgesetzt werden können. Als Primärprodukte entstehen Milchsäuresalze und Wasserstoff, als Nebenprodukt gegebenenfalls 1,2-Propandiol. In Einzelfällen werden jedoch mitunter auch andere Nebenprodukte mit ein bis sechs Kohlenstoffatomen beobachtet, beispielhaft werden Mehrfachalkohole mit und ohne Säurefunktionen, beispielsweise Gluconsäure, oder andere sogenannte Fruchtsäuren oder Glykolsäure gefunden. Alle Produkte können jedoch stofflichen oder energetischen Verwertungspfaden zugeführt werden.

Der chemische Reaktionsverlauf ist insofern überraschend, als vielmehr zu erwarten gewesen wäre (vgl. z. B. US 5 367 112 A), daß ein Polyalkohol, wie z. B. Sorbitol, nur an einer oder an beiden primären Alkoholgruppen zu einer Carbonsäure oxidiert wird. Überraschend wurde jedoch gefunden, daß Polyole, wie Glycerin und Sorbitol, sowie deren Oligomere, wie z. B. Polyglycerine, unter Veränderung oder Spaltung der Ausgangsmoleküle quantitativ in Milchsäure, gegebenenfalls 1,2-Propandiol als Nebenprodukt und Wasserstoff umgewandelt werden können. Dieses stellt eine bedeutsame Besonderheit der vorliegenden Erfindung dar.

Es ist bekannt, daß Polyethylenglykol nur an endständigen Alkoholgruppen zu einer Carbonsäure oxidiert wird, wobei die Ethergruppen im Molekül jedoch dabei erhalten bleiben (vgl. z. B. PCT/EP 97/05224 entsprechend der WO 98/13140 A1). Es wurde nunmehr überraschenderweise gefunden, daß im Rahmen des erfindungsgemäßen Verfahrens die Etherbindungen von Polyglycerinen, welche aus einem komplexen Gemisch von linearen, zyklischen und verzweigten Glycerinethern bestehen können, durch das erfindungsgemäße Verfahren gespalten werden. Als Produkte finden sich nur Milchsäure und Wasserstoff mit dem Nebenprodukt 1,2-Propandiol.

Unerwarteterweise wurde auch gefunden, daß wäßrige Lösungen von Zukkern, wie z. B. Sucrose (Rübenzucker) und Polysaccharide, wie z. B. Stärke, bei den vorgenannten Temperaturen unter alkalischen Bedingungen und vorzugsweise Wasserstoffatmosphäre stabil sind und an einem Katalysator, insbesondere an einem Kupfer- oder Kupfer/Zirconium-Katalysator, eine Spaltung der Ausgangsmoleküle in Milchsäure, gegebenenfalls 1,2-Propandiol als Nebenprodukt und Wasserstoff stattfindet.

Ohne sich auf eine bestimmte Theorie festlegen zu wollen, findet unter den zuvor angegebenen Bedingungen ein komplizierter Abbau der Di- und Polysaccharide, gepaart mit deren Spaltung in Monosaccharide, statt. Diese sind bekannterweise hydrierbar, im wesentlichen zu den Produkten Mannitol und Sorbitol, welche wiederum mit dem erfindungsgemäßen Verfahren zu Milchsäure und gegebenenfalls 1,2-Propandiol (Nebenprodukt) umwandelbar sind. Überraschenderweise gelingt diese Umwandlung bei den vorgenannten Temperatur- und Druckbereichen.

Gemäß einer bevorzugten Ausführungsform der vorliegenden Erfindung kann insbesondere wie folgt vorgegangen werden: Die Reaktion kann beispielsweise in gerührten Standarddruckreaktoren durchgeführt werden. Alternativ ist die Reaktionsführung in Festbettreaktoren (z. B. *Trickle-Bed*) oder Slurry-Reaktoren (z. B. Blasensäule) möglich. Im folgenden wird die Reaktionsführung rein beispielhaft und nicht beschränkend in einem Rührreaktor beschrieben. Ein nichtaktivierter Katalysator der vorgenannten Art wird in den Reaktor überführt. Die Aktivierung des Katalysators erfolgt beispielsweise bei 150 bis 300°C, bevorzugt 180 bis 250°C, im Wasserstoffstrom bei einem Absolutdruck von 1 bis 5 bar, bevorzugt 2 bis 3 bar. Die Rohstoffe, Natronlauge und Wasser werden nach der Aktivierung des Katalysators in den Reaktor eingeleitet. Das molare Verhältnis von Base, vorzugsweise NaOH, KOH, Ca(OH)₂ etc. oder auch anderer organischer und anorganischer Basen, berechnet als OH⁻, zu C₃-Einheiten des bzw. der Edukte beträgt vorteilhafterweise etwa 2 : 1, bevorzugt etwa 1,5 : 1, besonders bevorzugt etwa 1,1 : 1, jedoch mindestens 0,1 : 1. Der Reaktor ist vorzugsweise unter Inertgasdruck oder Wasserstoffdruck von beispielsweise 5 bis 15 bar zu setzen. Unter Rühren wird der Reaktorinhalt auf eine Reaktionstemperatur von beispielsweise 100 bis 200°C gebracht. Nach Einsetzen der Reaktion steigt der Reaktordruck aufgrund der Wasserstofffreisetzung an. Der Reaktordruck von beispielsweise 1 bis 20 bar, bevorzugt 5 bis 15 bar, besonders bevorzugt 10 bis 12 bar, wird über ein Regelventil kontrolliert. Das entstandene Wasserstoffgas wird aufgefangen und gegebenenfalls aufgereinigt oder getrocknet. Die Reaktionszeit beträgt 0,5 bis 8 Stunden, bevorzugt 1 bis 4 Stunden, besonders bevorzugt 1,5 bis 2 Stunden. Das Ende der Reaktion ist am asymptotisch abfallenden Druckgradienten zu erkennen. Nach der Reaktionszeit wird der Reaktorinhalt gekühlt und abgezogen, der Katalysator wird abgefiltert und in den Reaktor zur Wiederverwendung zurückgeführt.

Das erfindungsgemäße Verfahren ist mit einer Vielzahl von Vorteilen verbunden, von denen nachfolgend in nicht beschränkender Weise nur einige aufgezeigt werden sollen.

Das erfindungsgemäße Verfahren eröffnet Pfade zur stofflichen und energetischen Nutzung nachwachsender Rohstoffe. Mit der vorliegenden Erfindung lassen sich somit regenerative Einsatzstoffe mit an sich geringem Wertschöpfungspotential in Chemikalien, insbesondere Milchsäure bzw. Lactate und hieraus herstellbare Biopolymere (Polylactide), mit hohem Marktwert umwandeln.

Die aus den Salzen (Lactate) ableitbare Milchsäure stellt eine zukünftig relevante, aus Biomasse ableitbare Grundchemikalie dar. Besonders geeignet ist Milchsäure als Monomer für Kunststoffe (Polymilchsäure bzw. Polylactide) oder als Grundbaustein für Lösungsmittel oder oberflächenaktive Substanzen. Lactate finden darüber hinaus auch in verschiedensten anderen Produkten Anwendung, so beispielsweise in Kosmetika, Nahrungsmitteladditiven, Agrochemikalien oder dergleichen.

Das erfindungsgemäße Verfahren generiert im Fall von Polyalkoholen und Zuckern als Edukte gleichermaßen biogenen Wasserstoff, welcher wiederum für energetische Anwendungen, chemische Hydrierreaktionen, als Treibstoff oder dergleichen genutzt werden kann - in Kombination mit der stofflichen Nutzung der eingesetzten Biomasse unter optimaler Nutzung der vorhandenen natürlichen Syntheseleistung ("Bioraffineriekonzept").

Die in einem Verfahren zusammen mit den Polyolen oder Zuckern eingesetzten Chemikalien zur Erzeugung der freien Milchsäure (wäßriges Alkali und anorganische oder organische Säuren oder saure Ionenaustauscher) verlassen den Prozeß als Salze, die beispielsweise mittels Elektrodialyse wieder in den Prozeß zurückgeführt werden können; für den Fall, daß beispielsweise Salzsäure eingesetzt wird, erlaubt die Einbeziehung einer Chlor-Alkali-Elektrolyse in den Stoffkreislauf eine Rückführung des gebildeten Kochsalzes in den Prozeß und stellt daher eine bevorzugte Variante dar.

Gegenüber der derzeit industriell angewandten fermentativen Route der Milchsäureherstellung zeigt die neuartige, erfindungsgemäße chemische Prozeßvariante eine sehr viel höhere Raum-Zeit-Ausbeute. Während die Produktivität der Milchsäurefermentation mit Raum-Zeit-Ausbeuten von 5 bis 30 g/(l · h) charakterisiert wird, sind mit dem erfindungsgemäßen Verfahren deutlich höhere Raum-Zeit-Ausbeuten beispielsweise von 30 bis 200 g/(l · h) möglich.

Die vorliegende Erfindung ermöglicht sowohl die energetische als auch die stoffliche Nutzung nachwachsender Rohstoffe. Mit dem erfindungsgemäßen Verfahren können insbesondere öl-, zucker- und stärkehaltige Rohstoffe umgewandelt werden, wie zuvor beschrieben, insbesondere Glycerine, wie 1,2,3-Propantriol und dessen Oligomere (Polyglycerine), sowie Mono-, Di- und Polysaccharide, wie Stärke oder Cellulose.

Das erfindungsgemäße katalytische Verfahren wandelt diese aus nachwachsenden Rohstoffen ableitbaren Einsatzstoffe bzw. Edukte mit guter bis hoher Selektivität und hoher Raum-Zeit-Ausbeute in die chemischen Grundstoffe 2-Hydroxypropansäure (Milchsäure) bzw. dessen Salze um, wobei mitunter 1,2-Propandiol als Nebenprodukt entstehen kann, welches aber wiederum weiter zu Milchsäure umgesetzt werden kann.

Wie zuvor beschrieben, läßt sich das erfindungsgemäße katalytische Verfahren auf primäre Alkohole wie auch auf Aldehyde und insbesondere auch auf mehrwertige Alkohole und deren Oligomere anwenden. Gewisse Etherbindungen der Ausgangssubstanzen werden mit dem erfindungsgemäßen Verfahren überraschenderweise gespalten, so daß eine selektive Umsetzung zu den Produkten 2-Hydroxypropansäure (Milchsäure) und Wasserstoff sowie gegebenenfalls 1,2-Propandiol als Nebenprodukt erfolgt.

Im Rahmen des erfindungsgemäßen Verfahrens werden Übergangsmetallkatalysatoren eingesetzt, insbesondere Zirconium und/oder Kupfer und gegebenenfalls weitere Metalle enthaltende Katalysatoren, wobei die Katalysatoren grundsätzlich entweder als Vollkatalysatoren bzw. Katalysatoren in Masse (z. B. Raney-Kupfer) oder als geträgerte Katalysatoren (z. B. Kupfer auf Trägermaterial, wie z. B. Kupfer, insbesondere Cu⁰, auf Zirconiumoxid als Trägermaterial, d. h. Cu⁰Zr^{IV}O₂) eingesetzt werden können.

Die Besonderheit des erfindungsgemäßen Verfahrens liegt insbesondere darin, daß kostengünstige Produkte (beispielsweise entsäuertes Rohglycerin, d. h. von Fett, Fettsäureestern und von freier Fettsäure befreites Glycerin, beispielsweise aus der Biodieselherstellung oder der Oleochemie bzw. Fettspaltung, und Aufschlüsse zuckerbasierter Rohstoffe, wie z. B. Sorbitol und Mannitol sowie Zuckergemische aus Glucose, Maltose und Fructose, wie auch Polysaccharide, mit hoher Ausbeute direkt zu den Wertprodukten umgesetzt werden können. Als Primärprodukte entstehen Milchsäure bzw. Milchsäuresalze und Wasserstoff; als Nebenprodukt wird insbesondere 1,2-Propandiol erhalten, welches sich weiter zu Milchsäure bzw. Milchsäuresalzen umsetzen läßt. In Einzelfällen sind jedoch auch andere Nebenprodukte mit eins bis sechs Kohlenstoffatomen möglich, beispielhaft sind Mehrfachalkohole mit und ohne Säurefunktionen, beispielsweise Gluconsäure, andere sogenannte Fruchtsäuren oder Glykolsäure zu nennen. Alle Produkte können stofflichen oder energetischen Verwertungspfaden zugeführt werden.

Die vorliegende Erfindung wird anhand der nachfolgenden Ausführungsbeispiele veranschaulicht, welche die vorliegende Erfindung jedoch keinesfalls beschränken sollen.

### AUSFÜHRUNGSBEISPIELE:

### Allgemeine Herstellvorschrift für den eingesetzten Katalysator

Zirconiumoxychloridoctahydrat wird in Wasser gelöst, wobei die Wassermenge im Bereich von 400 ml bis 700 ml, bevorzugt 500 ml bis 600 ml, besonders bevorzugt etwa 550 ml, Wasser je 100 mmol Zirconiumoxychloridoctahydrat variieren kann. Durch nachfolgende Zugabe von 25%iger wäßriger Natronlauge wird Zirconiumhydroxid gefällt, bis ein pH-Wert von 7 erreicht wird. Zur Zirconiumhydroxidsuspension wird eine Lösung von Kupfernitrattrihydrat in Mengen von 50 mmol bis 90 mmol, bevorzugt 65 bis 80 mmol, besonders bevorzugt etwa 75 mmol, je 100 mmol Zirconiumhydroxid in je 100 ml Wasser und von Calciumnitrattetrahydrat in Mengen von 0,3 mmol bis 0,6 mmol, bevorzugt 0,4 bis 0,6 mmol, besonders bevorzugt etwa 0,5 mmol, je 100 mmol Zirconiumhydroxid in je 30 ml Wasser je 1 mmol Calciumnitrattetrahydrat hinzugefügt und die entsprechenden Hydroxide anschließend durch Zugabe von weiterer 25%iger wäßriger Natronlauge ausgefällt, bis ein pH-Wert von 10,5 erreicht wird. Die Suspension wird abfiltriert und der Feststoff anschließend viermal jeweils mit 220 ml Wasser je 100 mmol Zirconiumhydroxid gewaschen. Der Feststoff wird in 30 Minuten auf 500 °C aufgeheizt und insgesamt 3 Stunden bei 500 °C geglüht.

### Beispiel 1

Das Beispiel illustriert die mit dem erfindungsgemäßen Verfahren durchführbare Umsetzung von Glycerin zu Milchsäure unter gleichzeitiger Bildung von Wasserstoff mit 1,2-Propandiol als Nebenprodukt. In einem druckfesten 2-Liter-Rührbehälter werden 48,5 g des zuvor hergestellten Katalysators vorgelegt. Der Reaktor wird mit Stickstoff inertisiert und anschließend mit Wasserstoff beaufschlagt. Der Überdruck beträgt 1 bar. Bei einer Rührerdrehzahl von 100 Umdrehungen pro Minuten (U/min bzw. rpm) wird der Reaktor auf 200 °C bis 230 °C aufgeheizt. Die Aktivierung wird über zwei Stunden durchgeführt. Anschließend wird der Reaktor abgekühlt. Glycerin (158,4 g bzw. 1,7 mol), NaOH (69,3 g bzw. 1,7 mol) und Wasser (287,3 g) werden zugefügt. Ein Wasserstoffüberdruck von 5 bar wird angelegt. Der Reaktorinhalt wird unter Rühren auf die Reaktionstemperatur von 170 °C aufgeheizt. Die Temperatur wird über zwei Stunden konstantgehalten. Der entstehende Wasserstoff wird sukzessiv abgeführt, so daß der Reaktorüberdruck 12 bar nicht überschreitet. Im Produkt werden 1,3 mol Milchsäure und 0,3 mol 1,2-Propandiol analysiert. Der Umsatz ist größer als 94 %, die Milchsäureselektivität ist größer als 80 Mol-%, bezogen auf Glycerin. Das als Nebenprodukt entstandene 1,2-Propandiol kann nachfolgend rezykliert und weiter zu Milchsäure umgesetzt werden.

### Beispiel 2

Das Beispiel illustriert die mit dem Verfahren durchführbare Umsetzung von Polyglycerinen, im besonderen des Triglycerins zu Milchsäure unter gleichzeitiger Bildung von Wasserstoff mit 1,2-Propandiol als Nebenprodukt. In einem druckfesten 2-Liter-Rührbehälter werden 51,7 g des zuvor hergestellten Katalysators vorgelegt. Die Aktivierung des Katalysators erfolgt wie in Beispiel 1. Nach dem Abkühlen des Reaktors werden Triglycerin (175,6 g bzw. 0,7 mol), NaOH (76,9 g bzw. 1,9 mol) und Wasser (318,6 g) zugefügt. Das eingesetzte Triglycerin ist von technischer Qualität und enthält mehr als 80 Gew.-% Triglycerin und weniger als 20 Gew.-% Di- und Tetraglycerin. Ein Wasserstoffüberdruck von 5 bar wird angelegt. Der Reaktorinhalt wird unter Rühren auf die Reaktionstemperatur von 150 °C aufgeheizt. Die Temperatur wird über zwei Stunden konstantgehalten. Der entstehende Wasserstoff wird sukzessiv abgeführt, so daß der Reaktorüberdruck 12 bar nicht überschreitet. Im Produkt werden 1,8 mol Milchsäure, 0,2 mol 1,2-Propandiol und 0,05 mol Glycerin analysiert. Der Umsatz ist größer als 95 %, die Milchsäureselektivität ist größer als 85 Mol-%, bezogen auf Triglycerin. Das als Nebenprodukt entstandene 1,2-Propandiol kann nachfolgend rezykliert und weiter zu Milchsäure umgesetzt werden.

### Beispiel 3

Das Beispiel illustriert die mit dem Verfahren durchführbare Umsetzung von Polyglycerinen, im besonderen eines Gemisches aus linearen, verzweigten und cyclischen Polyglycerinen, zu den Produkten Milchsäure und Wasserstoff mit 1,2-Propandiol als Nebenprodukt. In einem druckfesten 2-Liter-Rührbehälter werden 30,0 g des zuvor hergestellten Katalysators vorgelegt. Die Aktivierung des Katalysators erfolgt wie in Beispiel 1. Nach dem Abkühlen des Reaktors werden Polyglycerin (121,5 g), NaOH (74,8 g bzw. 1,9 mol) und Wasser (254,0 g) zugefügt. Polyglycerin kann durch Glycerinkondensation unter alkalischen Bedingungen synthetisiert werden (vgl. z. B. DE 41 17 033 A1). Dafür werden in einem gerührten Reaktionsgefäß mit Rückflußkühler, Wasserabscheider und Innenthermometer 736 g (8 mol) Glycerin (99,5gew.-%ig) vorgelegt und mit 17,6 g (0,32 mol) Kaliumhydroxid versetzt. Die Reaktionsmischung wird in einer Stickstoffatmosphäre auf 240 °C erhitzt, wobei das Kondensationswasser über den Wasserabscheider entfernt wird. Die Reaktion wird abgebrochen, nachdem die für die Bildung von Diglycerin erforderliche Wassermenge abgeschieden ist. Ein Wasserstoffüberdruck von 8 bar wird angelegt. Der Reaktorinhalt wird unter Rühren auf die Reaktionstemperatur von 170 °C aufgeheizt. Die Temperatur wird über drei Stunden konstantgehalten. Der entstehende Wasserstoff wird sukzessiv abgeführt, so daß der Reaktorüberdruck 12 bar nicht überschreitet. Im Produkt werden 92 g Milchsäure analysiert. Die Ausbeute ist größer als 75 % (massenbezogen).

### Beispiel 4

Das Beispiel illustriert die mit dem Verfahren durchführbare Umsetzung von Zuckeralkoholen, im besonderen von Sorbitol, zu den Produkten Milchsäure, 1,2-Propandiol als Nebenprodukt und Wasserstoff. In einem druckfesten 2-Liter-Rührbehälter werden 30,0 g des zuvor hergestellten Katalysators vorgelegt. Die Aktivierung des Katalysators erfolgt wie in Beispiel 1. Nach dem Abkühlen des Reaktors werden Sorbitol (122,3 g bzw. 0,7 mol), NaOH (62,7 g bzw. 1,6 mol) und Wasser (265,7 g) zugefügt. Ein Wasserstoffüberdrock von 8 bar wird angelegt. Der Reaktorinhalt wird unter Rühren auf die Reaktionstemperatur von 165 °C aufgeheizt. Die Temperatur wird über eine Stunde konstantgehalten. Der entstehende Wasserstoff wird sukzessive abgeführt, so daß der Reaktorüberdruck 12 bar nicht überschreitet. Im Produkt werden 0,7 mol Milchsäure und 0,04 mol 1,2-Propandiol analysiert. Die Ausbeute ist größer als 51 % (massenbezogen). Weiterhin findet sich im Produkt eine nicht identifizierte Substanz, welche anhand der HPLC-Retentionszeit Gluconsäure zugeordnet werden kann. Der Umsatz an Sorbitol ist größer als 99 %, die Milchsäureselektivität ist größer als 50 Mol-%, bezogen auf Sorbitol. Das als Nebenprodukt entstandene 1,2-Propandiol kann nachfolgend rezykliert und weiter zu Milchsäure umgesetzt werden.

### Beispiel 5

Das Beispiel illustriert die mit dem Verfahren durchführbare Umsetzung von Disacchariden, im besonderen von Sucrose, zu den Produkten Milchsäure und Wasserstoff, wobei 1,2-Propandiol nur in Spuren gebildet wird. In einem druckfesten 2-Liter-Rührbehälter werden 7,0 g des zuvor hergestellten Katalysators vorgelegt. Die Aktivierung des Katalysators erfolgt wie in Beispiel 1. Nach dem Abkühlen des Reaktors werden Sucrose (52,9 g bzw. 0,2 mol), NaOH (33,1 g bzw. 0,8 mol) und Wasser (164,4 g) zugefügt. Ein Wasserstoffüberdruck von 8 bar wird angelegt. Der Reaktorinhalt wird unter Rühren auf die Reaktionstemperatur von 170 °C aufgeheizt. Die Temperatur wird über fünf Stunden konstantgehalten. Der entstehende Wasserstoff wird sukzessiv abgeführt, so daß der Reaktorüberdruck 14 bar nicht überschreitet. Im flüssigen Produkt werden 0,4 mol Milchsäure analysiert. Weiterhin findet sich im Produkt eine nicht identifizierte Substanz, welche anhand der HPLC-Retentionszeit Gluconsäure zugeordnet werden kann. Der Umsatz an Sucrose ist größer als 99 %, und die Milchsäureselektivität ist größer als 57 Mol-%, bezogen auf Sucrose.

## Patentansprüche

1. Verfahren zur Herstellung von 2-Hydroxypropansäure (Milchsäure) oder deren Salzen (Lactaten), wobei wenigstens eine mindestens drei Kohlenstoffatome aufweisende organische Ausgangsverbindung, welche ausgewählt ist aus einem primären Alkohol der allgemeinen Formel (I') 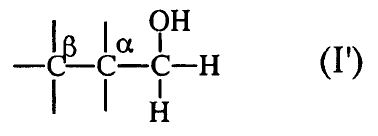 und/oder einem Aldehyd der allgemeinen Formel (I") 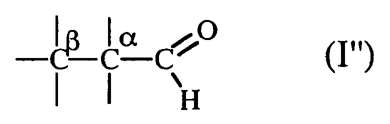 wobei der primäre Alkohol bzw. der Aldehyd eine Hydroxylgruppe in α-Position zur primären Alkoholfunktion bzw. zur Aldehydgruppe sowie eine Hydroxyl- oder Ethergruppe in β-Position zur primären Alkoholfunktion bzw. zur Aldehydgruppe aufweist,
in Gegenwart eines Übergangsmetallkatalysators zu 2-Hydroxypropansäure (Milchsäure) oder deren Salzen (Lactaten) umgesetzt wird,
wobei als Übergangsmetallkatalysator ein auf Kupfer in Form von Cu⁰ basierter Katalysator und/oder ein Kupfer in Form von Cu⁰ enthaltender Katalysator eingesetzt wird und
wobei das Verfahren unter einem Absolutdruck im Bereich von 0,1 bis < 20 bar und bei Temperaturen im Bereich von 80 bis 190 °C durchgeführt wird und wobei das Verfahren unter basischen Reaktionsbedingungen durchgeführt wird, wobei das molare Verhältnis von eingesetztem OH⁻ zu erzeugender Milchsäure bzw. Lactat mindestens 1 : 1 beträgt.

2. Verfahren nach Anspruch 1, **dadurch gekennzeichnet, daß** als Übergangsmetallkatalysator ein auf Kupfer in Form von Cu⁰ basierter und/oder Kupfer in Form von Cu⁰ enthaltender Katalysator in Kombination mit mindestens einem weiteren Metall, vorzugsweise in Kombination mit Zirconium, Cobalt, Nickel, Silber, Zink, Palladium, Ruthenium, Platin und/oder Rhodium, eingesetzt wird.

3. Verfahren nach Anspruch 1 oder 2, **dadurch gekennzeichnet, daß** als Übergangsmetallkatalysator Cu⁰Zr^{IV}O₂ eingesetzt wird.

4. Verfahren nach einem der vorangehenden Ansprüche, **dadurch gekennzeichnet, daß** der Katalysator in aktivierter Form eingesetzt wird, insbesondere wobei die Aktivierung unter Wasserstoffatmosphäre, insbesondere bei einem Absolutdruck von 1 bis 5 bar, vorzugsweise 2 bis 3 bar, durchgeführt wird.

5. Verfahren nach einem der vorangehenden Ansprüche, **dadurch gekennzeichnet, daß** das Verfahren in Gegenwart mindestens eines Promotors, insbesondere in Gegenwart eines Komplexbildners für die hergestellte 2-Hydroxypropansäure (Milchsäure) oder deren Salze (Lactate), vorzugsweise in Gegenwart eines Erdalkalimetalls, besonders bevorzugt Calcium und/oder Magnesium, durchgeführt wird, insbesondere wobei der Promotor mit dem Katalysator kombiniert vorliegt, insbesondere hierauf aufgebracht ist, und/oder insbesondere wobei der Promotor in der Reaktionslösung befindlich ist.

6. Verfahren nach einem der vorangehenden Ansprüche, **dadurch gekennzeichnet, daß** für den Fall, daß als Ausgangsverbindung der primäre Alkohol der allgemeinen Formel (I') eingesetzt wird, die Reaktion unter gleichzeitiger Dehydrogenierung erfolgt, insbesondere wobei pro 1 Mol erzeugter 2-Hydroxypropansäure (Milchsäure) oder deren Salz (Lactat) 1 Mol Wasserstoff generiert wird.

7. Verfahren nach einem der vorangehenden Ansprüche, **dadurch gekennzeichnet, daß** die Ausgangsverbindung mindestens drei Kohlenstoffatome oder ein Mehrfaches hiervon, insbesondere zwei, drei, vier oder mehr C3-Einheiten, aufweist, insbesondere wobei im Fall von Ausgangsverbindungen von mehr als einer C3-Einheit die Reaktion unter Molekülspaltung erfolgt.

8. Verfahren nach einem der vorangehenden Ansprüche, **dadurch gekennzeichnet, daß** das molare Verhältnis von eingesetztem OH⁻ zu erzeugender Milchsäure bzw. Lactat insbesondere im Bereich von 1 : 1 bis 10 : 1, vorzugsweise 1,05 : 1 bis 5 : 1, besonders bevorzugt 1,1 : 1 bis 2 : 1, ganz besonders bevorzugt 1,1 : 1 bis 1,5 : 1, variiert.

9. Verfahren nach einem der vorangehenden Ansprüche, **dadurch gekennzeichnet, daß** das Verfahren mit einer Selektivität in bezug auf Milchsäure bzw. Lactat, berechnet als prozentuales Verhältnis der gebildeten Molmenge Milchsäure bzw. Lactat zu der eingesetzten Molmenge an Ausgangsverbindung, von mindestens 50 Mol-%, insbesondere mindestens 55 Mol-%, vorzugsweise mindestens 60 Mol-%, durchgeführt wird.

10. Verfahren nach einem der vorangehenden Ansprüche, **dadurch gekennzeichnet, daß** die Ausgangsverbindung der allgemeinen Formel (Ia) 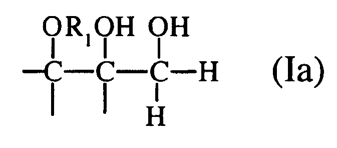 oder der allgemeinen Formel (Ib) 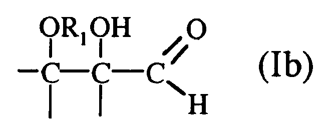 entspricht, wobei in den Formeln (Ia) und (Ib) der Rest R₁, unabhängig voneinander, jeweils Wasserstoff oder einen organischen Rest, vorzugsweise Wasserstoff, bezeichnet,
wobei der organische Rest ausgewählt sein kann aus einem oder mehreren der folgenden Reste:
• einer Alkylgruppe;
• einer Arylgruppe;
• einer Arylalkylgruppe;
• einer Alkylarylgruppe;
• einer polyalkoholischen Gruppe (Polyolylgruppe), insbesondere ausgewählt aus (i) Mono-, Di- oder Poly-1,2,3-propantriolylgruppen (Mono-, Di- oder Polyglyceringruppen), vorzugsweise linearen, verzweigten und/oder cyclischen Mono-, Di- oder Polyglyceringruppen mit 1 bis 10 Glycerineinheiten, oder (ii) Resten, insbesondere C3- oder C6-Resten, von Zuckern oder Zuckerderivaten, vorzugsweise auf Hexosebasis.

11. Verfahren nach einem der vorangehenden Ansprüche, **dadurch gekennzeichnet, daß** die Ausgangsverbindung der allgemeinen Formel (Ia') 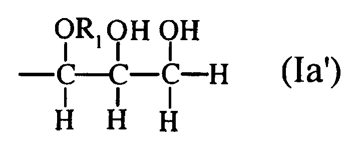 oder der allgemeinen Formel (Ib') 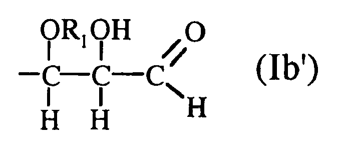 entspricht, wobei in den Formeln (Ia') und (Ib') der Rest R₁ die zuvor angegebene Bedeutung hat.

12. Verfahren nach einem der vorangehenden Ansprüche, **dadurch gekennzeichnet, daß** die Ausgangsverbindung der allgemeinen Formel (Ia") 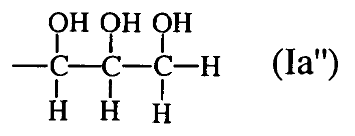 oder der allgemeinen Formel (Ib") 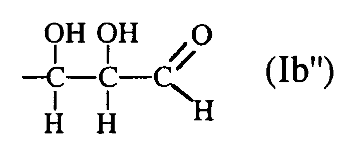 entspricht.

13. Verfahren nach einem der vorangehenden Ansprüche, **dadurch gekennzeichnet, daß** die Ausgangsverbindung der allgemeinen Formel 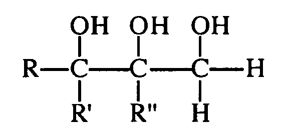 entspricht, wobei die Reste R, R' und R", jeweils unabhängig voneinander, Wasserstoff; Alkyl; gegebenenfalls substituiertes Aryl, insbesondere Phenyl, Hydroxyphenol und Alkoxyphenyl, insbesondere Methoxyphenyl; Arylalkyl; Alkylaryl; Halogen, vorzugsweise Fluor, bezeichnen.

14. Verfahren nach einem der vorangehenden Ansprüche, **dadurch gekennzeichnet, daß** die Ausgangsverbindung der allgemeinen Formel 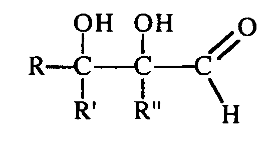 entspricht, wobei die Reste R, R' und R", jeweils unabhängig voneinander, Wasserstoff; Alkyl; gegebenenfalls substituiertes Aryl, insbesondere Phenyl, Hydroxyphenol und Alkoxyphenyl, insbesondere Methoxyphenyl; Arylalkyl; Alkylaryl; Halogen, vorzugsweise Fluor, bezeichnen.

15. Verfahren nach einem der vorangehenden Ansprüche, **dadurch gekennzeichnet, daß** die Ausgangsverbindung ausgewählt ist aus
(i) 1,2,3-Propantriol (Glycerin) und dessen linearen, verzweigten oder cyclischen Oligomeren (Oligoglycerinen) und Polymeren (Polyglycerinen);
(ii) Glycerinaldehyd (2,3-Hydroxypropionaldehyd);
(iii) Zuckern und Zuckeralkoholen, insbesondere Mono- und Disacchariden, bevorzugt auf Basis von Hexosen, ganz besonders bevorzugt Sucrose (Saccharose), Glucose, Fructose, Galactose, Lactose und Maltose, sowie daraus ableitbaren Zuckeralkoholen, insbesondere Sorbitol und Mannitol;
(iv) Polysacchariden, insbesondere auf Basis von Hexosen, bevorzugt Stärke und Cellulose;
sowie deren Gemischen.

## Claims

1. A method for preparing 2-hydroxypropanoic acid (lactic acid) or salts (lactates) thereof, wherein at least one organic starting compound containing at least three carbon atoms, said starting compound being selected from a primary alcohol of general formula (I') 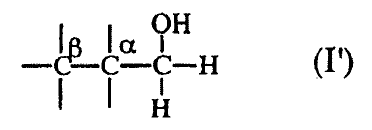 and/or from an aldehyde of general formula (I") 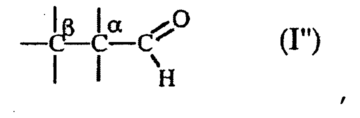 wherein the primary alcohol or the aldehyde has a hydroxyl group in the α-position to the primary alcohol function or to the aldehyde group as well as a hydroxyl group or ether group in the β-position to the primary alcohol function or to the aldehyde group,
is reacted in the presence of a transition metal catalyst to form 2-hydroxypropanoic acid (lactic acid) or salts (lactates) thereof,
wherein a catalyst based on copper in the form of Cu⁰ and/or a catalyst containing copper in the form of Cu⁰ is used as the transition metal catalyst, and
wherein the method is carried out at an absolute pressure in the range of 0.1 to < 20 bar and at temperatures in the range of 80 to 190 °C, and wherein the method is carried out under alkaline reaction conditions, wherein the molar ratio of OH⁻ used to lactic acid or lactate produced is at least 1:1.

2. The method according to claim 1, **characterised in that** a catalyst based on copper in the form of Cu⁰ and/or containing copper in the form of Cu⁰ is used as the transition metal catalyst in combination with at least one further metal, preferably in combination with zirconium, cobalt, nickel, silver, zinc, palladium, ruthenium, platinum and/or rhodium.

3. The method according to claim 1 or 2, **characterised in that** Cu⁰Zr^{IV}O₂ is used as the transition metal catalyst.

4. The method according to one of the preceding claims, **characterised in that** the catalyst is used in activated form, the activation process being carried out in particular in a hydrogen atmosphere, in particular at an absolute pressure of 1 to 5 bar, preferably 2 to 3 bar.

5. The method according to one of the preceding claims, **characterised in that** the method is carried out in the presence of at least one promoter, in particular in the presence of a complexing agent for the 2-hydroxypropanoic acid (lactic acid) or salts (lactates) thereof produced, preferably in the presence of an alkaline earth metal, particularly preferably calcium and/or magnesium, in particular the promoter being combined with the catalyst, in particular applied thereto, and/or in particular the promoter being located in the reaction solution.

6. The method according to one of the preceding claims, **characterised in that**, if the primary alcohol of general formula (I') is used as the starting compound, the reaction is carried out with simultaneous dehydrogenation, in particular 1 mol of hydrogen being generated per 1 mol of 2-hydroxypropanoic acid (lactic acid) or salt (lactate) thereof produced.

7. The method according to one of the preceding claims, **characterised in that** the starting compound contains at least three carbon atoms or a multiple thereof, in particular two, three, four or more C3 units, the reaction being carried out in particular with molecular cleavage in the case of starting compounds of more than one C3 unit.

8. The method according to one of the preceding claims, **characterised in that** the molar ratio of OH⁻ used to lactic acid or lactate produced varies in particular in the range of 1:1 to 10:1, preferably 1.05:1 to 5:1, more preferably 1.1:1 to 2:1, most preferably 1.1:1 to 1.5:1.

9. The method according to one of the preceding claims, **characterised in that** the method is carried out with a selectivity with regard to lactic acid or lactate, calculated as a ratio (in %) of the molar amount of lactic acid or lactate formed to the molar amount of starting compound used, of at least 50 mol %, in particular at least 55 mol %, preferably at least 60 mol %.

10. The method according to one of the preceding claims, **characterised in that** the starting compound corresponds to general formula (Ia) 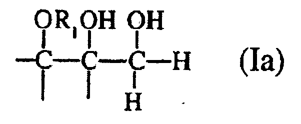 or general formula (Ib) 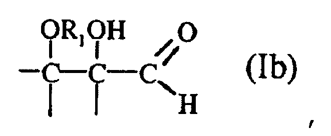 wherein, in formulas (Ia) and (Ib), the radical R₁, independently of one another, denotes hydrogen or an organic radical in each case, preferably hydrogen, wherein the organic radical can be selected from one or more of the following radicals:
• an alkyl group;
• an aryl group;
• an arylalkyl group;
• an alkylaryl group;
• a polyalcohol group (polyol group), in particular selected from (i) mono-, di-, or poly-1,2,3-propanetriolyl groups (mono-, di- or polyglycerol groups), preferably linear, branched and/or cyclic mono-, di- or polyglycerol groups containing 1 to 10 glycerol units, or (ii) radicals, in particular C3 or C6 radicals, of sugars or sugar derivatives, preferably based on hexose.

11. The method according to one of the preceding claims, **characterised in that** the starting compound corresponds to general formula (Ia') 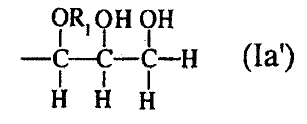 or general formula (Ib') 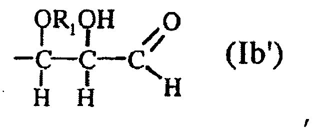 wherein, in formulas (Ia') and (Ib'), the radical R₁ has the meaning as disclosed above.

12. The method according to one of the preceding claims, **characterised in that** the starting compound corresponds to general formula (Ia") 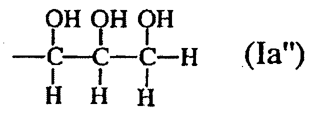 or general formula (Ib")

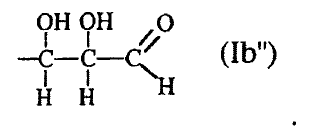

13. The method according to one of the preceding claims, **characterised in that** the starting compound corresponds to general formula 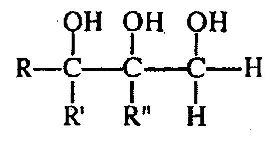 wherein the radicals R, R' and R", independently of one another, denote hydrogen; alkyl; optionally substituted aryl, in particular phenyl, hydroxyphenol and alkoxyphenol, in particular methoxyphenol; arylalkyl; alkylaryl; halogen, preferably fluorine.

14. The method according to one of the preceding claims, **characterised in that** the starting compound corresponds to general formula 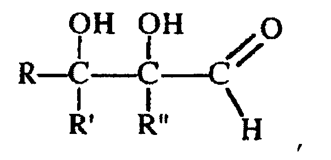 wherein the radicals R, R' and R", independently of one another, denote hydrogen; alkyl; optionally substituted aryl, in particular phenyl, hydroxyphenol and alkoxyphenol, in particular methoxyphenol; arylalkyl; alkylaryl; halogen, preferably fluorine.

15. The method according to one of the preceding claims, **characterised in that** the starting compound is selected from
(i) 1,2,3-propanetriol (glycerol) and the linear, branched or cyclic oligomers (oligoglycerols) and polymers (polyglycerols) thereof;
(ii) glycerolaldehyde (2,3-hydroxypropionic aldehyde);
(iii) sugars and sugar alcohols, in particular mono- and disaccharides, preferably based on hexoses, most preferably sucrose (saccharose) glucose, fructose, galactose, lactose and maltose, as well as sugar alcohols derivable therefrom, in particular sorbitol and mannitol;
(iv) polysaccharides, in particular based on hexoses, preferably starch and cellulose;
and mixtures thereof.

## Revendications

1. Procédé de production d'acide 2-hydroxypropanoïque (acide lactique) ou de ses sels (lactates), au moins un composé initial organique présentant au moins trois atomes de carbone, lequel est choisi parmi un alcool primaire de la formule générale (I') 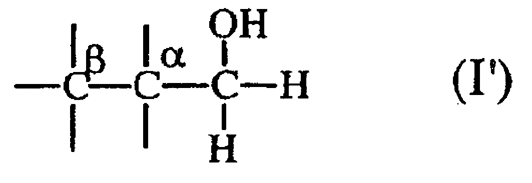 et ou un aldéhyde de la formule générale (I"), 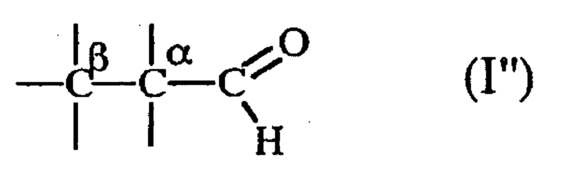 l'alcool primaire ou l'aldéhyde présentant un groupe hydroxyle en position α par rapport à la fonction alcoolique primaire ou au groupe aldéhyde ainsi qu'un groupe hydroxyle ou groupe éther en position β par rapport à la fonction alcoolique primaire ou au groupe aldéhyde,
étant transformé en présence d'un catalyseur de métal transitoire en acide 2-hydroxypropanoïque (acide lactique) ou en ses sels (lactates),
en tant que catalyseur de métal transitoire étant utilisé un catalyseur basé sur le cuivre sous forme de Cu⁰ et/ou un catalyseur contenant du cuivre sous forme de Cu⁰ et
le procédé étant réalisé sous une pression absolue de l'ordre de 0,1 à < 20 bar et à des températures de l'ordre de 80 à 190 °C et le procédé étant réalisé sous des conditions de réaction basiques, le rapport moléculaire du OH' utilisé à l'acide lactique ou aux lactates à créer étant d'au moins 1 : 1.

2. Procédé selon la revendication 1, **caractérisé en ce qu'**en tant que catalyseur de métal transitoire, il est utilisé un catalyseur basé sur le cuivre sous forme de Cu⁰ et/ou un catalyseur contenant du cuivre sous forme de Cu⁰ en association avec au moins un métal supplémentaire, de préférence en association avec du zirconium, du cobalt, du nickel, de l'argent, du zinc, du palladium, du ruthénium, du platine et/ou du rhodium.

3. Procédé selon la revendication 1 ou la revendication 2, **caractérisé en ce qu'**en tant que catalyseur de métal transitoire, il est utilisé du Cu⁰Zr^{IV}O₂.

4. Procédé selon l'une quelconque des revendications précédentes, **caractérisé en ce que** le catalyseur est utilisé dans sa forme activée, notamment l'activation étant réalisée sous atmosphère hydrogénée, notamment à une pression absolue de 1 à 5 bar, de préférence de 2 à 3 bar.

5. Procédé selon l'une quelconque des revendications précédentes, **caractérisé en ce que** le procédé est réalisé en présence d'au moins un promoteur, notamment en présence d'un agent complexant pour l'acide 2-hydroxypropanoïque (acide lactique) produit ou ses sels (lactates), de préférence en présence d'un métal alcalinoterreux, de manière particulièrement préférée de calcium et/ou de magnésium, notamment le promoteur étant présent en association avec le catalyseur, étant notamment appliqué sur ce dernier et/ou notamment le promoteur se trouvant dans la solution réactive.

6. Procédé selon l'une quelconque des revendications précédentes, **caractérisé en ce que** dans le cas où on utilise en tant que composé initial l'alcool primaire de la formule générale (I'), la réaction s'effectue sous déshydrogénation simultanée, notamment 1 mole d'hydrogène étant généré pour 1 mole d'acide 2-hydroxypropanoïque (acide lactique) créé ou de son sel (lactate).

7. Procédé selon l'une quelconque des revendications précédentes, **caractérisé en ce que** le composé initial présente au moins trois atomes de carbone ou un multiple de cette quantité, notamment deux, trois, quatre unités C3 ou plus, notamment dans le cas de composés initiaux de plus d'une unité C3, la réaction s'effectuant sous division moléculaire.

8. Procédé selon l'une quelconque des revendications précédentes, **caractérisé en ce que** le rapport moléculaire du OH' utilisé à l'acide lactique à créer ou lactate varie notamment de l'ordre de 1 : 1 à 10 : 1, de préférence de l'ordre de 1,05 : 1 à 5 : 1, de manière particulièrement préférée, de l'ordre de 1,1 : 1 à 2 : 1, de manière tout particulièrement préférée, de l'ordre de 1,1 : 1 à 1,5 : 1.

9. Procédé selon l'une quelconque des revendications précédentes, **caractérisé en ce que** le procédé est réalisé avec une sélectivité par rapport à l'acide lactique ou au lactate calculée en tant que taux de la quantité moléculaire d'acide lactique ou lactate par rapport à la quantité moléculaire de composé initiale d'au moins 50 % en moles, notamment d'au moins 55 % en moles, de préférence d'au moins 60 % en moles.

10. Procédé selon l'une quelconque des revendications précédentes, **caractérisé en ce que** le composé initial correspond à la formule générale (Ia) 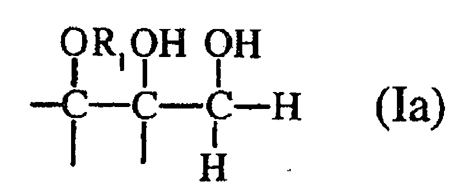 ou à la formule générale (Ib) 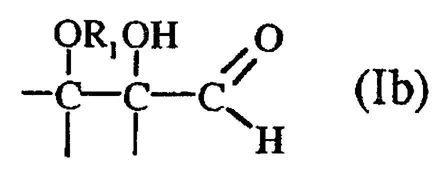 dans les formules (Ia) et (Ib), le radical R1 désignant, indépendamment l'un de l'autre, chaque fois de l'hydrogène ou un radical organique, de préférence de l'hydrogène,
le radical organique pouvant être choisi parmi un ou plusieurs des radicaux suivants :
• un groupe alkyle ;
• un groupe aryle ;
• un groupe arylalkyle ;
• un groupe alkylaryle
• un groupe polyalcoolique (groupe polyol), notamment choisi parmi les groupes (i) mono-, di- ou poly-1,2,3-propantriolyle (groupes mono-, di- ou polyglycérol) de préférence les groupes mono-, di- ou polyglycérol linéaires, ramifiés et/ou cycliques avec de 1 à 10 unités de glycérol ou (ii) radicaux, notamment des radicaux en C3 ou C6 de sucres ou de dérivés de sucre, de préférence sur base de hexose.

11. Procédé selon l'une quelconque des revendications précédentes, **caractérisé en ce que** le composé initial correspond à la formule générale (Ia') 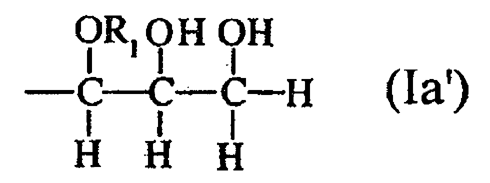 ou à la formule générale (Ib') 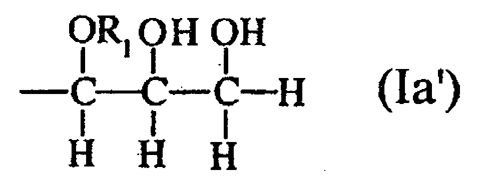 dans les formules (Ia') et (Ib'), le radical R₁ ayant la signification précédemment indiquée.

12. Procédé selon l'une quelconque des revendications précédentes, **caractérisé en ce que** le composé initial correspond à la formule générale (Ia") 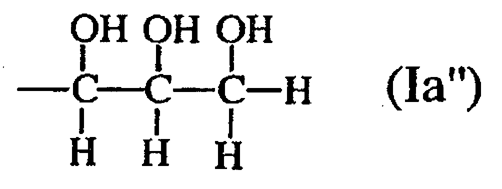 ou à la formule générale (Ib")

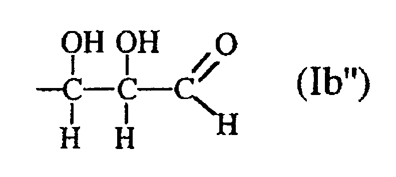

13. Procédé selon l'une quelconque des revendications précédentes, **caractérisé en ce que** le composé initial correspond à la formule générale 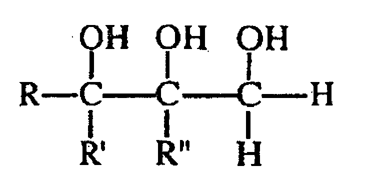 les radicaux R, R' et R" désignant chaque fois indépendamment l'un de l'autre de l'hydrogène, de l'alkyle ; le cas échéant de l'aryle substitué, notamment du phényle, de l'hydroxyphénol et de l'alcoxyphényle, notamment du métoxyphényle ; de l'arylalkyle : de l'alkylaryle ; de l'halogène, de préférence du fluor.

14. Procédé selon l'une quelconque des revendications précédentes, **caractérisé en ce que** le composé initial correspond à la formule générale 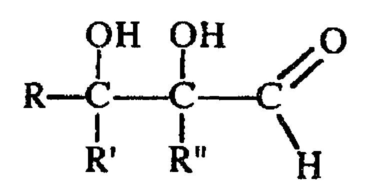 les radicaux R, R' et R" désignant chaque fois indépendamment l'un de l'autre de l'hydrogène, de l'alkyle ; le cas échéant de l'aryle substitué, notamment du phényle, de l'hydroxyphénol et de l'alcoxyphényle, notamment du métoxyphényle ; de l'arylalkyle : de l'alkylaryle ; de l'halogène, de préférence du fluor.

15. Procédé selon l'une quelconque des revendications précédentes, **caractérisé en ce que** le composé initial est choisi parmi
(i) le 1,2,3-propantriol (glycérol) et ses oligomères linéaires, ramifiés ou cycliques (oligoglycérines) et polymères (polyglycérines) ;
(ii) le glycérolaldéhyde (2,3-hydroxypropionaldéhyde) ;
(ii) les sucres et les alcools de sucre, notamment des monosaccarides et des disaccarides, de préférence sur la base d'hexoses, de manière particulièrement préférée, sur la base de sucrose (saccharose), de glucose, de fructose, de galactose, de lactose et de maltose, ainsi que des alcools de sucre pouvant en être dérivés, notamment le sorbitol et le mannitol ;
(iv) les polysaccarides, notamment sur base d'hexoses, de préférence, l'amidon et la cellulose ;
ainsi que leurs mélanges.
